Europäisches Patentamt

**European Patent Office**

Office européen des brevets

(19)

(11) Veröffentlichungsnummer: **0 072 978**
**B1**

(12)

# EUROPÄISCHE PATENTSCHRIFT

(45) Veröffentlichungstag der Patentschrift:
16.07.86

(51) Int. Cl.⁴: **C 12 N 9/58** // C12R1/845

(21) Anmeldenummer: **82107321.0**

(22) Anmeldetag: **12.08.82**

(54) Verfahren zur Herstellung saurer Protease.

(30) Priorität: **20.08.81 DE 3132936**

(43) Veröffentlichungstag der Anmeldung:
**02.03.83 Patentblatt 83/9**

(45) Bekanntmachung des Hinweises auf die Patenterteilung:
**16.07.86 Patentblatt 86/29**

(84) Benannte Vertragsstaaten:
**AT BE CH DE FR GB IT LI LU NL SE**

(56) Entgegenhaltungen:
**DE - A - 2 528 490**
**DE - A - 2 755 126**
**US - A - 3 151 039**
**US - A - 3 212 905**

**CHEMICAL ABSTRACTS, Band 91, Nr. 13, 24. September 1979, Seiten 470,471, Nr. 106568k, Columbus Ohio (USA); YUNG NOK LEE et al.: "Studies on the enzyme activities of Rhizopus distributed in South Korea. I. On the amylase, protease and cellulase activities".**
**H. RUTTLOFF et al. in INDUSTRIELLE ENZYME (1979) 1. Aufl. DARMSTADT, STEINKOPFF VERLAG S. 152-158**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Schindler, Joachim, Dr., Am Eichkamp 158, D-4010 Hilden (DE)**
Erfinder: **Bartnik, Friedhelm, Dr., Melanchthonstrasse 11, D-4000 Düsseldorf (DE)**
Erfinder: **Schmid, Rolf, Dr., Am Nettchesfeld 13, D-4000 Düsseldorf (DE)**
Erfinder: **Weiss, Albrecht, Dr., Amselweg 8, D-4006 Erkrath (DE)**

**Beschreibung**

Gegenstand der Patentanmeldung DE-OS 2 528 490 ist ein Verfahren zur Herstellung einer sauren Protease mit breitem Wirkungsbereich durch Züchten eines aus Erdproben isolierten Pilzstammes der Spezies Rhizopus rhizopodiformis. Der Pilzstamm besitzt die Hinterlegungsnummer CBS 227.75 des Centraal Bureau voor Schimmel-Cultures in Baarn (Niederlande).

Die Erfinder haben sich die Aufgabe gestellt, ein neues Verfahren bereitzustellen, das es erlaubt, Proteasen von Stämmen der Rhizopus rhizopodiformis mit verbesserten Ausbeuten zur Verfügung zu stellen.

Gegenstand der Erfindung ist somit ein Verfahren zur Herstellung einer sauren Protease mit breitem pH-Wirkungsbereich in hoher Ausbeute durch aerobes Züchten eines Pilzstammes der Gattung Rhizopus, dadurch gekennzeichnet, dass man Mutanten des Wildstammes Rhizopus rhizopodiformis CBS 227.75 mit der Hinterlegungsbezeichnung

Rhizopus rhizopodiformis III-34 (CBS 219.80)
Rhizopus rhizopodiformis III-46 (CBS 220.80)
Rhizopus rhizopodiformis III-59 (CBS 221.80)
Rhizopus rhizopodiformis III-65 (CBS 222.80)

in einem Nährmedium, das assimilierbare Kohlenstoff- und Stickstoffquellen enthält, bei pH-Werten zwischen 3 und 7 und Temperaturen zwischen 25 und 50 °C züchtet und das erzeugte Enzym abtrennt.

Die genannten Mutanten leiten sich von dem Wildstamm Rhizopus rhizopodiformis CBS 225.75 ab. Die Isolierung der Mutanten erfolgte in folgender Weise:

Auf Würzebouillonagar versporte Schrägkulturen des Wildstammes wurden mit steriler 0,005%iger Natriumlaurylsulfatlösung abgeschwemmt, die Sporensuspension durch eine sterile Glasfritte (D-3) zur Abtrennung der Myzelfragmente filtriert und anschliessend 15 Minuten bei 6000 U/Min. zentrifugiert. Die sedimentierten Sporen wurden in sterilem 0,1 M Acetatpuffer (pH 4,5) aufgenommen und die Sporenkonzentration unter dem Mikroskop auf ca. $10^8$ Keime/ml eingestellt. Die eingestellte Sporenlösung wurde anschliessend mit einer UV-Lampe (Wellenlänge 254 nm, 13 Watt) bestrahlt bis zu einer Abtötungsrate von 99,9%. Die bestrahlten Sporen wurden auf Caseinatagarplatten folgender Zusammensetzung ausgespatelt:

| | |
|---|---|
| 0,10% Sojamehl | 0,02% $CaCl_2 \cdot 2H_2O$ |
| 0,10% Maisquellwasser | 0,40% Casein |
| 0,50% Glukose | 1,60% Agar |
| 0,50% Gelatine | pH-Wert 5,5 |
| 0,50% Malzextrakt | |
| 0,10% Na-Desoxycholat | |
| 0,24% $KH_2PO_4$ | |
| 0,10% $MgCl_2 \cdot 6H_2O$ | |
| 0,05% $MnSO_4 \cdot 4H_2O$ | |

Die Platten wurden 2–3 Tage bei 30 °C inkubiert. Nur Kolonien mit verstärkter caseolytischer Hofbildung wurden anschliessend als Stammkulturen isoliert und weiter untersucht.

Die neuen Stämme wurden zur Überprüfung ihrer proteolytischen Syntheseleistungen in Schüttelkulturen bei 30 °C kultiviert (500 ml Erlenmeyerkolben mit Schikanen, Schüttelfrequenz 150 U/Min.). Die Zusammensetzung des Mediums war wie folgt:

0,25% $KH_2PO_4$
0,06% $NaNO_3$
2,00% Casein
0,80% Gelatine
0,50% Sojamehl
0,50% Malzextrakt
2,00% Maisstärke (amylolytisch abgebaut)
10,00% Vol % 20%iger Weizenkleieextrakt
10,00% Vol % 10%iger Haferflockenextrakt
pH 5,5

Hierbei wurden aus dem Wildstamm eine Reihe von Mutantenstämmen isoliert, die deutlich höhere Proteaseaktivitäten bildeten. Von dem aktivsten dieser Stämme ausgehend wurden dann erneut Mutationsversuche eingeleitet, die nochmals zu Stämmen mit gesteigerten Aktivitäten führten. Diese Versuche wurden schliesslich noch ein 3. Mal durchgeführt und insgesamt folgende Stämme isoliert und die Proteaseausbeute bestimmt.

Tabelle 1

| Stämme | Generation | Protease-Aktivität (mTU/ml) |
|---|---|---|
| Wildstamm CBS 227.75 | | 8 |
| Stamm I–261 | 1 | 15 |
| Stamm I–266 | 1 | 18 |
| Stamm II–47 | 2 | 20 |
| Stamm III–34 | 3 | 37 |
| Stamm III–44 | 3 | 37 |
| Stamm III–46 | 3 | 32 |
| Stamm III–59 | 3 | 28 |
| Stamm III–65 | 3 | 21 |

Von diesen wurden die folgenden Stämme im Central Bureau voor Schimmelcultures in Baarn (Niederlande) hinterlegt:

Tabelle 2

| Stamm | Hinterleg.-Nr. | Hinterleg.-Tag |
| --- | --- | --- |
| III–34 | CBS 219.80 | 03.04.80 |
| III–46 | CBS 220.80 | 03.04.80 |
| III–59 | CBS 221.80 | 03.04.80 |
| III–65 | CBS 222.80 | 03.04.80 |

Die von den Stämmen der Tabelle 1 gebildete Protease besitzt die gleichen Eigenschaften wie diejenige des Wildstammes CBS 227.75. Sie zeichnet sich durch ein besonders breites Wirkungsspektrum im schwach sauren Bereich zwischen pH 2,5 bis 6,5 aus. Das Wirkungs-Optimum liegt bei pH 4,5, der Bereich der 80%igen Maximalaktivität bei pH 3,3 bis 5,9, der Bereich der 60%igen Maximalaktivität bei pH 3,0 bis 6,4. Die Protease eignet sich in besonderem Masse als Zusatzstoff für Tierfuttermittel, insbesondere zur Verbesserung der Ergebnisse bei der Aufzucht und Mast von Geflügel, Schweinen, Kälbern und Nutzfischen. Sie kann jedoch darüber hinaus auch für andere Einsatzzwecke, bei denen saure Proteasen verwendet werden können, angewendet werden, wie z.B. in Lebensmittel verarbeitenden Betrieben, in sauren Wasch- und Reinigungsmitteln, insbesondere Reinigungsmitteln für Kacheln, Fussböden und Tische, in Krankenhäusern und im Haushalt, als Lederhilfsmittel in der Gerberei, ferner in hoch gereinigter Form im medizinischen Anwendungsbereich als Digestivum.

Das Verfahren zur Herstellung der Protease kann durch Züchten der selektierten Mutanten in einem flüssigen oder festen Nährmedium durchgeführt werden, wobei das flüssige Nährmedium im allgemeinen bevorzugt wird. Bei Züchtung in einer Nährlösung wird nach dem üblichen aeroben Schüttel-Kultur- oder Fermentationsverfahren gearbeitet.

Der erfindungsgemäss zu verwendende Nährboden wird in üblicher Weise hergestellt und soll eine Kohlenstoffquelle, eine Stickstoffquelle und andere von dem Mikroorganismus benötigte Nähr- und Wuchsstoffe enthalten. Als geeignete Kohlenstoffquellen sind Stärke, Dextrin, Rohrzukker, Glukose, Fruktose, Maltose und zuckerhaltige Abfallstoffe zu nennen. Als Stickstoffquellen kommen Ammoniumsalze, Harnstoff, Casein, Gelatine, Maisquellwasser und Sojabohnenmehl bzw. -kuchen in Frage. Weiterhin können anorganische Salze, wie beispielsweise Natrium-, Kalium-, Ammoniumhydrogenphosphate, Calcium- und Magnesiumsalze dem Nährboden zugesetzt werden. Ferner kann es vorteilhaft sein, Wuchsstoffe, wie z.B. Hefeextrakt und Vitamine dem Nährboden zuzusetzen.

Die Fermentationstemperatur kann sich in den Grenzen von 25°C bis 50°C bewegen, ist aber vorzugsweise zwischen 27 bis 32°C zu wählen. Der pH-Wert des Nährbodens kann 3,0 bis 7,0 betragen, vorzugsweise 4,0 bis 6,0. Die Züchtung wird im allgemeinen in einer Zeit von 20 bis 96 Stunden durchgeführt.

Die nach dem erfindungsgemässen Verfahren hergestellte Protease kann aus der filtrierten oder zentrifugierten Nährlösung nach üblichen Methoden durch Zusatz organischer Lösungsmittel oder durch Aussalzen mit z.B. Natriumsulfat oder Ammoniumsulfat ausgefällt und konzentriert werden. Eine Reinigung lässt sich durch Dialyse-Verfahren oder durch Behandlung an Ionenaustauscherharzen bewerkstelligen.

Die Feststellung der proteolytischen Aktivität der vorliegenden Protease erfolgte nach dem bekannten Prinzip der Bestimmung nach Anson: eine geeignet verdünnte Menge Enzymlösung wird bei 40°C 20 Minuten mit einem gleichen Volumen einer 1,2%igen Caseinlösung inkubiert, wobei diese 0,6% Milchsäure, 6 Mol Harnstoff und 0,1 Mol Zitronen- oder Essigsäure enthält. Der pH-Wert der Caseinlösung wird durch Zusatz von 2 N Natronlauge auf 4,5 eingestellt. Nach der Inkubation wird im Volumenverhältnis 1:1 mit 0,4 N Trichloressigsäure versetzt, der sich bildende Niederschlag von unverdautem Casein abfiltriert und im Filtrat die beim Abbau entstandenen Protein-Spaltstücke nach einer beliebigen Eiweissbestimmungsmethode ermittelt. Geeignet hierfür ist z.B. das von Layne in Methods of Enzymology 3 (1957) Seiten 448 ff. beschriebene Verfahren.

Für jede Messprobe muss ein Blindwert angefertigt werden, bei dem zuerst Trichloressigsäure und dann Caseinlösung zugesetzt werden. Dieser Blindwert gibt neben dem Reagenzien-Leerwert den Anteil an niedermolekularen Peptiden an, der bereits vor der Verdauung in der Enzymlösung vorhanden ist. Die Differenz zwischen Haupt- und Blindwert wird bei der angegebenen Methode dann mit der Extinktion verglichen, die eine bestimmte Menge Tyrosin bei dieser Bestimmung liefert. Diese Menge Tyrosin ist dann ein Mass für die proteolytische Aktivität des vorliegenden Enzyms: eine Enzymeinheit (TU) ist diejenige Menge Enzym, die den gleichen Extinktionsunterschied zwischen Haupt- und Blindwert pro Minute verursacht wie eine 1 M Tyrosinlösung, die anstatt der Enzymlösung eingesetzt wird.

Die Messung der proteolytischen Aktivität bei höheren und tieferen pH-Werten als 4,5 ist durch passende Einstellung der Caseinlösung ohne weiteres möglich, wobei man jedoch den Essigsäurezusatz günstigerweise durch Zitronensäure ersetzt.

Beispiel 1

Zur Bereitung des Nährmediums wurden in 1 l Wasser 3 g Sojamehl, 3 g Maisquellwasser, 15 g Casein, 7 g Gelatine, 2,4 g $KH_2PO_4$, 0,5 g $MgSO_4 \cdot 7 H_2O$, 0,1 g $MnCl_2 \cdot 4 H_2O$, 0,1 g $CaCl_2 \cdot 2 H_2O$ und

20 g Maisstärke gelöst bzw. dispergiert. Der pH-Wert der Nährlösung betrug 5,3. Die Maisstärke wurde mittels Amylase weitestgehend abgebaut. In die sterilisierte Nährlösung wurden Sporen des Mutanten III-34 eingeimpft und die Kultur unter optimaler Belüftung bei 30 °C etwa 50 Stunden im Schüttelinkubator gezüchtet. Nach dieser Zeit wurde das Pilzmycel abfiltriert und die mycelfreie Kulturbrühe zur Bestimmung der Proteaseaktivität gemäss vorgenannten Verfahren verwendet. Dabei ergab sich, dass die Kulturlösung eine enzymatische Aktivität 37 mTU/ml erreichte.

Beispiel 2

Zur Bereitung des Nährmediums wurden in 1 l Leitungswasser 10 g Sojamehl (entölt), 5 g Maisquellwasser, 12 g Casein, 5 g Gelatine, 5 g Getreidetrockenschlempe, 2,4 g KH$_2$PO$_4$, 1 g NaNO$_3$, 1 g NH$_4$Cl, 0,01 g FeSO$_4$ und 30 g native Maisstärke gelöst bzw. dispergiert. Der pH-Wert der Nährlösung wurde nach dem Autoklavieren auf 5,3 eingestellt. Ein mit dieser Nährlösung angesetzter 1-Liter-Erlenmeyerkolben wurde mit 10 ml einer Czapek-Dox-Vorkultur (mit 5% Stärke und 0,5% Hefeextrakt) versetzt, die mit Sporen des Mutanten III-59 beimpft und 24 Stunden bei 30 °C geschüttelt worden war, unter Belüftung bei 30 °C etwa 72–96 Stunden betrieben, bis der pH-Wert auf 6,8 bis 7,0 angestiegen war. Danach wurde das Mycel abfiltriert und die klare Kulturbrühe zur Bestimmung der Proteaseaktivität gemäss vorgenanntem Verfahren verwendet. Dabei ergab sich, dass die Kulturlösung eine enzymatische Aktivität von 28 mTU/ml erreichte. Die Protease wurde isoliert durch Klarfiltration der Brühe aus 10 1 l Schüttelkolbenansätzen unter Zusatz von 5 g Filter Cel und 5 g Standard Super Cel der Firma Mansville, Einengen bei 50 Torr auf ein Drittel des Ausgangsvolumens und Ausfällen unter Zusatz von 39% wasserfreiem Natriumsulfat unter Rühren, wobei die Temperatur auf 38 – 40 °C gebracht wurde. Alternativ kann die Protease aber auch durch Zutropfen von 2 Volumina Ethanol, Methanol, Aceton oder anderer wassermischbarer Lösungsmittel bei einer Temperatur von –3 bis 5 °C ausgefällt werden. Der Niederschlag wurde abgesaugt und im Vakuum getrocknet.

Beispiel 3

Unter Anwendung des vorstehend geschilderten Bestimmungsverfahrens wurde die Aktivität der nach Beispiel 1 und 2 isolierten Protease in Abhängigkeit vom pH-Wert ermittelt. In der Tabelle sind die pH-Werte aufgeführt, bei denen die optimale bzw. 50% der beim optimalen pH-Wert gemessenen Aktivitäten vorliegen. Wie die Werte erkennen lassen, besitzen die Proteasen der in den Beispielen 1 und 2 verwendeten Mutanten den gleichen breiten und für die genannten Anwendungsgebiete günstigen pH-Wirkungsbereiche wie die aus der Wildform CBS 227.75 isolierte Protease.

Tabelle 3

| Enzym | pH–Optimum | 50 %-Werte |
|---|---|---|
| Protease aus Mutante III–34 | 4,0 – 4,5 | 2,5 – 6,5 |
| Protease aus Mutante III–59 | 4,0 – 4,5 | 2,5 – 6,5 |
| Protease aus CBS 227.75 | 4,0 – 4,5 | 2,5 – 6,5 |

Beispiel 4

Die aus den Mutanten Rhizopus rhizopodiformis III–34, III–46, III–59 und III–65 gewonnenen Proteasen wurden nach der Methode der Isoelektrofokussierung bzw. der Kreuzreaktion nach Ouchterlony mit der aus dem Wildstamm CBS 227.75 gewonnenen Protease verglichen. Hierbei ergab sich vollständige Identität sämtlicher Proteasen.

Tabelle 4

| | | Ergebnisse der Isoelektrofokussierung Proteinzone mit pI | | | | | | | | | | Immunologische Untersuchung Kreuzreaktion nach Ouchterlony mit CBS 227.25 |
|---|---|---|---|---|---|---|---|---|---|---|---|---|
| | | 4,7 | | 4,9 | | 5,1 | | 5,4 | | 6,1 | | |
| | | a | b | a | b | a | b | a | b | a | b | |
| Wildstamm CBS 227.25 | | X | X | XX | XX | XXX | XXX | XX | XX | XX | XX | + |
| Mutanten | III–34 | X | X | XX | XX | XXX | XXX | XX | XX | XX | XX | + |
| | III–46 | X | X | XX | XX | XXX | XXX | XX | XX | XX | XX | + |
| | III–59 | X | X | XX | XX | XXX | XXX | XX | XX | XX | XX | + |
| | III–65 | X | X | XX | XX | XXX | XXX | XX | XX | XX | XX | + |

a) Coomassie-blue Reaktion (Protein)
b) Zymogramm-Reaktion (Protease)

Methodenbeschreibung Isoelectrofoccussierung

Geräte: Multiphor 2117, LKB Instruments
LKB 2103 Power Supply

Material: LKB Ampholine PAG plates, LKB
Instruments pH 3,5–9,5
Anodenflüssigkeit: 1 M $H_3PO_4$
Kathodenflüssigkeit: 1 M NaOH

Isoelectrofoccussierung:

Nach dem Aufbringen des Polyacrylamidgels auf die wassergekühlte Apparatur werden mit Elektrodenlösung getränkte Papierelektrodenstreifen auf die Ränder des Gels gelegt. Auf der Mittellinie des Gels zwischen Anode und Kathode werden 5 mm breite Filterstreifen aufgebracht und 10 µl salzfreie Probelösung (5 mg/ml Protein) aufgetropft. Nach Verschliessen der Apparatur wird bei einer Stromstärke von 10 mA der pH-Gradient aufgebaut, nach 30 Min. die Probefilterstreifen entfernt und auf eine Stromstärke von 15 mA erhöht. Nach 2–2,5 Stunden ist die Trennung beendet.

Bestimmung der pH-Gradienten:

Der pH-Gradient wird durch eine kalibrierte Oberflächen-pH Elektrode dadurch bestimmt, dass man den pH-Verlauf zwischen Anode und Kathode auf mehreren Linien abgreift und ein pH/cm-Diagramm erstellt.

Fixierung und Färbung:

Fixierlösung: Zu 57,5 g Trichloressigsäure und 17,25 g Sulfosalicylsäure wird destilliertes Wasser gegeben und auf 500 ml aufgefüllt.

Entfärbelösung: Eine Mischung aus 500 ml Ethanol und 160 ml Essigsäure wird mit destilliertem Wasser zu 2000 ml verdünnt.

Färbelösung: 0,46 g Coomassie Blue G-250 in 400 ml Entfärbelösung.

Nach der Erstellung des pH-Profils wird das Gel 1 Stunde zur Proteinpräzipitation und Ampholin-Entfernung in gerührter Fixierlösung belassen, 5 Minuten mit Entfärbelösung gewaschen und anschliessend für 10 Minuten bei 60 °C in die Färbelösung gelegt. Das Gel wird durch mehrmaliges Wechseln der Entfärbelösung entfärbt, bis sich die blaugefärbten Proteinbanden deutlich vom Untergrund abheben.

Anhand des pH-Profils werden die isoelektrischen Punkte der Proteine bestimmt.

Methodenbeschreibung der Kreuzreaktion nach Ouchterlony

Material

Für die Kreuzreaktionen werden Immundiffusionsplatten der Firma Miles, Frankfurt eingesetzt (Code Nr. 64-276-1). Die Fertigplatten enthalten 0,9%ige Agarose in Borat/Kochsalzpuffer, pH 7,5 – Ionenstärke 0,175%. Die Platten enthalten ferner 0,01% Thiomersal als Bakteriostatikum und Trypanblau als Indikator für die proteinpräzipitationslinien.

Antikörper gegen die Protease CBS 227.75 wurden aus Kaninchen gewonnen.

Als positive Kontrolle diente Protease CBS 227.75 mit der die Immunisierung durchgeführt wurde.

Durchführung

In der Mitte der Diffusionsplatten werden 25 µl des Antiserums hineinpipettiert, in die aussen gelegenen Löcher jeweils 25 µl Proteaselösung bei einer Konzentration von 5 mg/ml bzw. 10 mg/ml.

Diffusionszeit: 72 Stunden bei 4 °C.

Präzipitationslinien können durch den in der Agarose enthaltenen Trypanblauindikator nachgewiesen werden. Die immunologische Kreuzreaktion fällt nur dann positiv aus, wenn die Antikörper erzeugende Protease mit der geprüften Protease identisch ist. Dies wird durch Verschmelzen der Präzipitationslinien festgestellt.

**Patentanspruch**

Verfahren zur Herstellung einer sauren Protease mit breitem pH-Wirkungsbereich in hoher Ausbeute durch aerobes Züchten eines Pilzstammes der Gattung Rhizopus, dadurch gekennzeichnet, dass man Mutanten des Wildstammes Rhizopus rhizopodiformis CBS 227.75 mit der Hinterlegungsbezeichnung

Rhizopus rhizopodiformis III–34 (CBS 219.80)
Rhizopus rhizopodiformis III–46 (CBS 220.80)
Rhizopus rhizopodiformis III–59 (CBS 221.80)
Rhizopus rhizopodiformis III–65 (CBS 222.80)

in einem Nährmedium, das assimilierbare Kohlenstoff- und Stickstoffquellen enthält, bei pH-Werten zwischen 3 und 7 und Temperaturen zwischen 25 und 50 °C züchtet und das erzeugte Enzym abtrennt.

**Claim**

A process for the preparation of an acidic protease active over a broad pH-range in a high yield by aerobic culture of a fungus strain of the species Rhizopus, characterized in that mutants of the wild strain Rhizopus rhizopodiformis CBS 227.75 deposited under the names

Rhizopus rhizopodiformis III–34 (CBS 219.80)
Rhizopus rhizopodiformis III–46 (CBS 220.80)
Rhizopus rhizopodiformis III–59 (CBS 221.80)
Rhizopus rhizopodiformis III–65 (CBS 222.80)

are cultured in a nutrient medium containing assimilable carbon and nitrogen sources at pH-values of from 3 to 7 and at temperatures of from 25 to 50 °C and the enzym produced is separated off.

## Revendication

Procédé pour l'obtention avec un rendement élevé d'une protéase acide active dans un large intervalle de pH, par culture aérobie d'une souche de moisissure du genre rhizopus, caractérisé par le fait que l'on cultive des mutants de la souche sauvage rhizopus rhizopodiformis CBS 227.75 ayant les désignations de dépôt

Rhizopus rhizopodiformis III–34 (CBS 219.80)
Rhizopus rhizopodiformis III–46 (CBS 220.80)
Rhizopus rhizopodiformis III–59 (CBS 221.80)
Rhizopus rhizopodiformis III–65 (CBS 222.80)
dans un milieu nutritif qui contient des sources de carbone et d'azote assimilables, à des pH compris entre 3 et 7 à des températures comprises entre 25 et 50 °C, et que l'on isole l'enzyme produite.